# EUROPEAN PATENT APPLICATION

(11) **EP 3 932 336 A1**
(43) Date of publication of application: **05.01.2022**
(21) Application number: 20799407.0
(22) Date of filing: 28.04.2020
(51) Int. Cl.: A61B 17/12

(54) **OCCLUDER WITH NO EXTERNALLY PROTRUDING STRUCTURE AT EITHER END**

(30) Priority: 29.04.2019 CN 201910355451; 29.04.2019 CN 201920611275 U
(71) Applicant: Mallow Medical (Shanghai) Co., Ltd., Xuhui District Shanghai 200231 (CN)
(72) Inventor: ZHANG, Jin, Shanghai 200231 (CN); ZHANG, Jian, Shanghai 200231 (CN); SUN, Kun, Shanghai 200232 (CN); WU, Hong, Shanghai 200232 (CN); MA, Caixia, Shanghai 200232 (CN)
(74) Representative: Haseltine Lake Kempner LLP
(86) International application number: PCT/CN2020/087406
(87) International publication number: WO 2020/221233

(57) **Abstract**

An occluder (1) with no externally protruding structure at either end, comprising a mesh body (10); a first closing line (20), the first closing line (20) passing through a plurality of grids formed by mesh at a first closing end (11) in order to implement closing of the first closing end (11); and a second closing line (30), the second closing line (30) passing through a plurality of grids formed by mesh at a second closing end (12) in order to implement closing of the second closing end (12). Compared to occluders in the prior art with a threaded connecting member, the present solution can reduce the manufacturing difficulty, reduce the reject rate, and save costs.

## Description

The application requests priorities of Chinese patent applications 201910355451X and 2019206112757, filed on April 29, 2019. The contents of above Chinese patent applications are incorporated herein by reference in their entirety.

### Technical Field

The invention relates to an occluder with no externally protruding structure at either end, to treat congenital heart diseases, including atrial septal defect, patent ductus arteriosus, ventricular septal defect, patent foramen ovale, diseases of the left atrial appendage, or blood vessels, and other occluding tissue lumens and the like which require occluding treatments.

### Background

In the prior art, one end of an occluder usually has a connector, the connector has internal threads to connect with a conveying equipment to implement the transport of the occluder. Since the manufacturing difficulty of the internal threads is relatively high, along with a relatively high reject rate during manufacturing processes, result in the waste on raw materials and the increase in ineffective labor cost. With the development of degradable occluder, since manufacturing a degradable occluder with a threaded connector is difficult to manufacture, so the method of this solution may be adopted to manufacture an occluder with no externally protruding structure at either end. While treating tumors and angiopathies, the occluder may also be implanted into the supplying blood vessel of diseased organs, occluding it, blocking the blood supply, expected to achieve the purpose of controlling the hemorrhage, treating tumors and angiopathies. The occluder may also be put into some tissue lumens, to close the tissue lumen, blocking the transfer of internal materials.

To sum up, in the prior art, it is difficult for manufacturing the occluder along with increased manufacturing cost because the connector of the occluder has threads.

### Content of the Present Invention

The technical problem to be solved in the present invention is overcoming the defects that in the prior art, it is difficult for manufacturing the occluder along with increased manufacturing cost because the connector of the occluder has threads, thus providing an occluder with no externally protruding structure at either end.

This present invention solves the above-mentioned technical problem by the following technical solution:

An occluder with no externally protruding structure at either end, wherein, it includes:
a mesh body, the mesh body has a first closing end and a second closing end disposed opposite to each other;
a first closing line, the first closing line passes through a plurality of grids formed by mesh filaments at a first closing end to implement closing of the first closing end;
a second closing line, the second closing line passes through a plurality of grids formed by mesh filaments at a second closing end to implement closing of the second closing end.

Preferably, both the first closing end and the second closing end have no externally protruding structure.

In this solution, both the first closing end and the second closing end have no externally protruding structure means: the external surface of the occluder where two closing ends of the occluder are located, is a flat and smooth surface. By using the above configuration, on one hand, the flat and smooth external surface of the occluder with no externally protruding structure at either end is easier for endothelial cells to climb onto, thus endothelialization will form rapidly on the surface of the occluder, reducing the time for endothelialization, which can also be considered as reducing the time needed for surgery recovery; on the other hand, the occluder with no externally protruding structure at either end does extremely minor damage to surrounding tissues.

Preferably, the second closing line has a circular closing line, the circular closing line passes through a plurality of grids formed by mesh filaments at a second closing end to implement closing of the second closing end, as a connector, the circular closing line connects to a conveying equipment which transports the occluder with no externally protruding structure at either end.

Preferably, the second closing line has a circular closing line and a transporting line, the circular closing line passes through a plurality of grids formed by mesh filaments at a second closing end to implement closing of the second closing end, two ends of the transporting line are respectively connected to the circular closing line to connect with a conveying equipment which transports the occluder with no externally protruding structure at either end.

By using the above configurations, transporting line may easily be jointed to the connecting member of the conveying equipment, which enables a much more reliable transport.

Preferably, materials of the mesh body, the first closing line, and the second closing line are all implantable materials, and are selected from at least one of the following materials:
(1) metals and their alloy materials, including stainless steel, cobalt-base alloys, titanium and titanium alloys, and Ni-Ti alloy shape memory materials;
(2) nondegradable bioinert medical polymer materials, including nylon, polyethylene terephthalate, polytetrafluoroethylene, extra-high molecular weight polyethylene, high-density polyethylene, polymethyl methacrylate, polypropylene, polycarbonate, polyurethane, organosilicon, and polyacrylonitrile;
(3) degradable biomedical materials, including polylactic acid, polyglycolide acid, polycaprolactone, polydioxanone, and the copolymer or the mixture of these materials.

There needs to be clarified, materials of the mesh body, the first closing line, and the second closing line may be selected from one or multiple materials under any subgroup of materials type (1) - (3), may also be selected from one or multiple materials under two or multiple subgroups of materials type (1) - (3).

Preferably, the mesh body is manufactured by methods of being woven from mesh filaments or 3D printing.

Preferably, the internal of the occluder with no externally protruding structure at either end is provided with fiber fabrics, non-woven fabrics or membranes.

By using the above configuration, fiber fabrics, non-woven fabrics or membranes may allow endothelial tissue cells to climb onto, and improve occlusion of blood or body fluid by apparatus.

Preferably, the occluder with no externally protruding structure at either end is: an atrial septal defect occluder, a patent ductus arteriosus occluder, a ventricular septal defect occluder, a patent foramen ovale occluder, a left atrial appendage occluder, or a vascular occluder.

Based on conformity with common knowledge of the field, each above-specified preferable condition, may be combined as will, which results in each preferable embodiment of this invention.

### Positive progressive effect of this invention are:

For occluder with no externally protruding structure at either end disclosed in this invention, two ends may both be closed by closing line into circular structures, closing line or transporting line may be captured by a conveying equipment to implement the transport of the occluder; compared to the occluder in the prior art with a threaded connector, the present solution can reduce the manufacturing difficulty, reduce the reject rate, and save costs. The occluder with no externally protruding structure at either end has a fine fit ability, may safely and effectively occlude heart defect or nonclosed places, may greatly reduce the residual split flow of postoperative ultrasonic examination, may also avoid or reduce attaching of platelet at exposing riveting points of traditional occluder with rivets at both ends, which reduce the risk of forming blood clots at portions of the occluder, where reduces the occurrence of occluder postoperative complications from the fundament. The occluder with no externally protruding structure at either end may also be used on treating tumors and angiopathy, the occluder is implanted into the supplying blood vessel of diseased organs, occluding it, blocking the blood supply, expected to achieve the purpose of controlling the hemorrhage, treating tumors and angiopathies. The occluder with no externally protruding structure at either end may also be put into some tissue lumens, to close the tissue lumens, blocking the transfer of internal materials.

### Brief description of the drawings

Figure 1 is a front view of the occluder with no externally protruding structure at either end in embodiment 1.
Figure 2 is a rear view of the occluder with no externally protruding structure at either end in embodiment 1.
Figure 3 is an enlarged view of the central section of Figure 1.
Figure 4 is a bottom view of the occluder with no externally protruding structure at either end in embodiment 1.
Figure 5 is a perspective view of the occluder with no externally protruding structure at either end in embodiment 1.
Figure 6 is a partial perspective view of the occluder with no externally protruding structure at either end at the second closing end in embodiment 2.

### Brief description of reference signs

- 1:: occluder with no externally protruding structure at either end
- 10:: mesh body
- 11:: first closing end
- 12:: second closing end
- 20:: first closing line
- 30:: second closing line
- 31:: circular closing line
- 32:: transporting line

### Detailed descriptions of the preferred embodiment

There is a preferred embodiment below, along with attached drawings to describe the invention more clearly.

### Embodiment 1

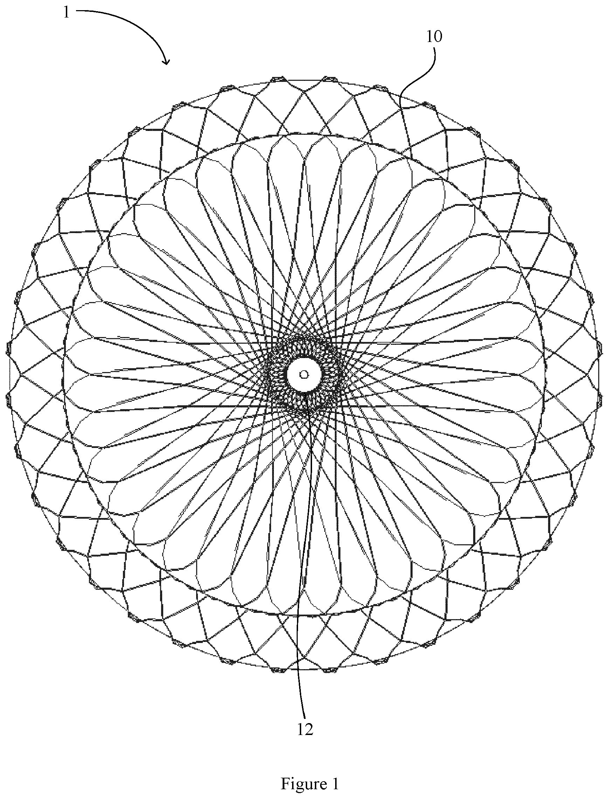

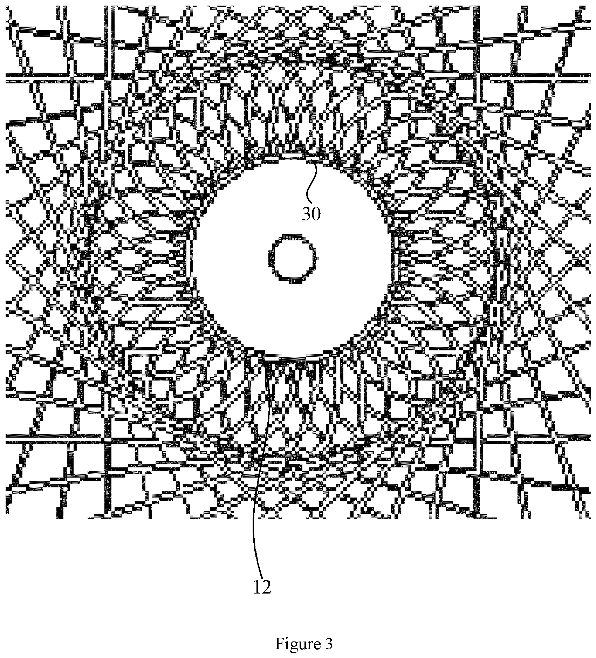

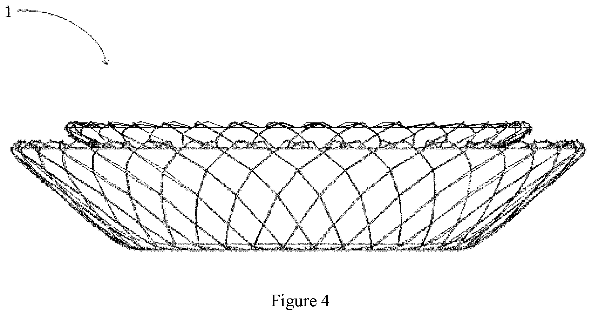

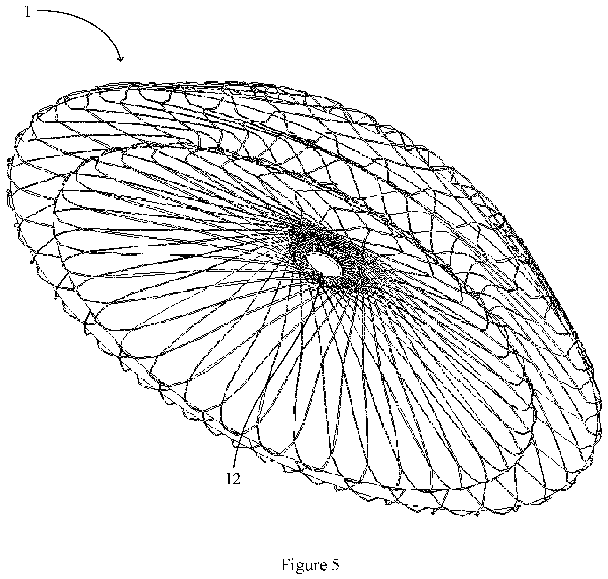

As shown in Figures 1-5, an occluder with no externally protruding structure at either end 1 includes: a mesh body 10, a first closing line 20, and a second closing line 30. The structure of the mesh body 10 is similar to the prior art, having two mesh plates, and internals of both mesh plates are stitched with membranes that allow the endothelial cells to climb onto. In the embodiment, both mesh plates are in concave form and having the same concave direction.

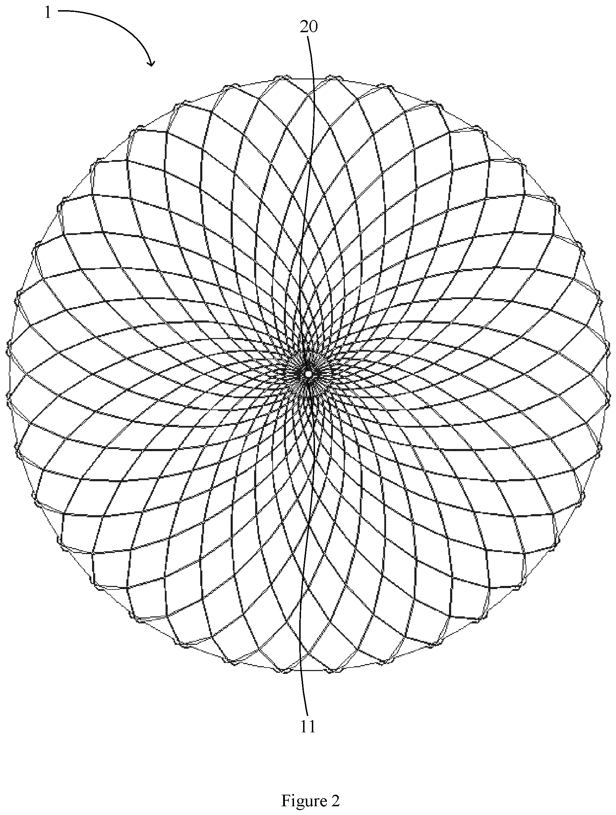

The mesh body 10 has a first closing end 11 and a second closing end 12 disposed opposite to each other, both the first closing end 11 and the second closing end 12 have no externally protruding structure. Shown at the front of Figure 1 is the second closing end 12, and shown at the front of Figure 2 is the first closing end 11. The first closing line 20 passes through a plurality of grids formed by mesh filaments at a first closing end 11 to implement closing of the first closing end 11. The second closing line 30 passes through a plurality of grids formed by mesh filaments at a second closing end 12 to implement closing of the second closing end 12. Wherein, the second closing line 30 has a circular closing line, passing through a plurality of grids formed by mesh filaments at a second closing end 12 to implement closing of the second closing end 12, as a connector, the circular closing line connects to a conveying equipment which transports the occluder with no externally protruding structure at either end.

In the embodiment, materials of the mesh body, the first closing line, and the second closing line are all implantable materials, in specific, are Ni-Ti alloy shape memory materials. In other alternative implementations, materials of the mesh body, the first closing line, and the second closing line may also use metals and their alloy materials, like stainless steels, cobalt-base alloys, and one or multiple types of titanium and titanium alloys, may still use nondegradable bioinert medical polymer materials, like one or multiple types of nylon, polyethylene terephthalate, polytetrafluoroethylene, extra-high molecular weight polyethylene, high-density polyethylene, polymethyl methacrylate, polypropylene, polycarbonate, polyurethane, organosilicon, and polyacrylonitrile, etc., may also use degradable biomedical materials, as one or multiple materials among polylactic acid, polyglycolide acid, polycaprolactone, and polydioxanone, or use the copolymer or the mixture of these materials.

To sum up, both ends are closed by the closing line into circular structures, wherein the closing line at proximate end may be captured by the conveying equipment to implement transport of the occluder, compared to occluders in the prior art with a threaded connector, the solution of this embodiment may reduce the risk at use, and increase the speed of endothelialization.

Additionally, both the first closing end and the second closing end have no externally protruding structure, on one hand, the flat and smooth external surface of the occluder with no externally protruding structure at either end is easier for endothelial cells to climb onto, thus endothelialization will form rapidly on the surface of the occluder, reducing the time for endothelialization, which can also consider as reducing the time needed for surgery recovery; on the other hand, the occluder with no externally protruding structure at either end does minor damage to surrounding tissues.

In the embodiment, the mesh body 10 is formed by woven from mesh filaments. In other alternative implementations, the mesh body 10 may also be manufactured by the 3D printing method.

Additionally, in the embodiment, the internal of the occluder with no externally protruding structure at either end is provided with membrane, which allows endothelial tissue cells to climb onto, and improve occlusion of blood or body fluid by apparatus. In other alternative implementations, the internal of the occluder with no externally protruding structure at either end may also be provided with fiber fabrics or non-woven fabrics, which allows endothelial tissue cells to climb onto, and improve occlusion of blood or body fluid by apparatus.

The occluder with no externally protruding structure at either end may be: a atrial septal defect occluder, a patent ductus arteriosus occluder, a ventricular septal defect occluder, a patent foramen ovale occluder, a left atrial appendage occluder, or a vascular occluder, may also be other medical apparatus for closing tissue lumen.

### Embodiment 2

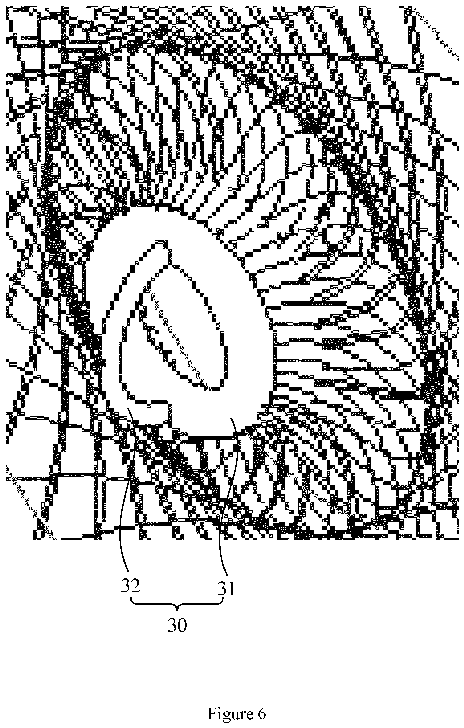

As shown in Figure 6, the embodiment 2 is similar to the embodiment 1, but in differences: in the embodiment 1, the second closing line 30 only includes the circular closing line, in the embodiment 2, however, the second closing line 30 has a circular closing line 31 and a transporting line 32. The circular closing line 31 passes through a plurality of grids formed by mesh filaments at a second closing end 12 to implement closing of the second closing end 12, two ends of the transporting line 32 are respectively connected to the circular closing line 31 to connect with a conveying equipment which transport the occluder with no externally protruding structure at either end 1.

The transporting line 32 may easily be jointed to the connecting member of the conveying equipment, which implements much more reliable transport.

Which needs to be clarified that, with regards to the structure of the transporting line 32, it may be configured into various structures according to actual needs, such as semicircular structures, U-shaped structure, etc., as long as the transporting line 32 can be connected to the circular closing line 31, to implement connection with the conveying equipment that transport occluder with no externally protruding structure at either end 1.

In Figure 6, the circular closing line 31 is shown thicker than the actual part for exemplary purpose.

In the description of the invention, one embodiment may include multiple figures, reference number of an identical component of an identical embodiment may not be noted on each figure, but the skilled in the art should understand that, while describing one or multiple figures, they may combine other figures for understanding, the skilled in the art should understand that, while words are not related to specific figures, they may combine all figures of the embodiment for the understanding.

In the description of this invention, which needs to understand that, terms like "up", "down", "front", "rear", "left", "right", "vertical", "horizontal", "top", "bottom", "internal", and "external" etc. which points directions or positioning relations out according to directions or positioning relations of the figures, are only for conveniently describe the invention and simplify the description, rather than referring or implying that the devices or components must have specific positions, be configured or operated in specific positions, thus it cannot be considered as qualifications/definitions of the invention.

Though detailed embodiments of the invention are described above, the skilled in the art should understand that, these embodiments are only exemplary descriptions, may apply various modifications or amendments onto these embodiments without deviating from the concept and essence of the invention. Thus, the scope of protection of this invention is defined by the attached claims.

## Claims

1. An occluder with no externally protruding structure at either end, wherein, it includes:
a mesh body, the mesh body has the first closing end and the second closing end disposed opposite to each other;
a first closing line, the first closing line passes through a plurality of grids formed by mesh filaments at a first closing end in order to implement closing of the first closing end;
a second closing line, the second closing line passes through a plurality of grids formed by mesh filaments at a second closing end in order to implement closing of the second closing end.

2. The occluder with no externally protruding structure at either end according to claim 1, wherein, both the first closing end and the second closing end have no externally protruding structure.

3. The occluder with no externally protruding structure at either end according to claim 1, wherein, the second closing line has a circular closing line, the circular closing line passes through a plurality of grids formed by mesh filaments at a second closing end to implement closing of the second closing end, as a connector, the circular closing line connects to a conveying equipment which transports the occluder with no externally protruding structure at either end.

4. The occluder with no externally protruding structure at either end according to claim 1, wherein, the second closing line has a circular closing line and a transporting line, the circular closing line passes through a plurality of grids formed by mesh filaments at a second closing end to implement closing of the second closing end, two ends of the transporting line are respectively connected to the circular closing line to connect with a conveying equipment which transport the occluder with no externally protruding structure at either end.

5. The occluder with no externally protruding structure at either end according to claim 1, wherein, materials of the mesh body, the first closing line, the second closing line are all implantable materials, and are selected from at least one of the following materials:
(1) metals and their alloy materials, including stainless steels, cobalt-base alloys, titanium and titanium alloys, Ni-Ti alloy shape memory materials;
(2) nondegradable bioinert medical polymer materials, including nylon, polyethylene terephthalate, polytetrafluoroethylene, extra-high molecular weight polyethylene, high density polyethylene, polymethyl methacrylate, polypropylene, polycarbonate, polyurethane, organosilicon, polyacrylonitrile;
(3) degradable biomedical materials, including polylactic acid, polyglycolide acid, polycaprolactone, polydioxanone, and copolymer or mixture of these materials.

6. The occluder with no externally protruding structure at either end according to claim 1, wherein, the mesh body is manufactured by methods of being woven from mesh filaments or 3D printing.

7. The occluder with no externally protruding structure at either end according to claim 1, wherein, the internal of the occluder with no externally protruding structure at either end is provided with fiber fabric, non-woven fabric or membrane.

8. The occluder with no externally protruding structure at either end according to any of claims 1-7, wherein, the occluder with no externally protruding structure at either end is: an atrial septal defect occluder, a patent ductus arteriosus occluder, a ventricular septal defect occluder, a patent foramen ovale occluder, a left atrial appendage occluder or a vascular occluder.
